# EUROPEAN PATENT APPLICATION

(11) **EP 3 729 972 A1**
(43) Date of publication of application: **28.10.2020**
(21) Application number: 18891472.5
(22) Date of filing: 05.12.2018
(51) Int. Cl.: A23L 33/11, A23L 33/105, A61K 36/886, A61P 3/10, A61P 29/00, A23G 3/34, A23G 3/48, A23L 2/38, A23L 2/52, A23L 21/15

(54) **METHOD OF PRODUCING ALOE POWDER**

(30) Priority: 19.12.2017 JP 2017242411
(71) Applicant: Morinaga Milk Industry Co., Ltd., Minato-ku Tokyo 108-8384 (JP)
(72) Inventor: OZAWA, Tomohito, Zama-shi, Kanagawa 252-8583 (JP); OCHI, Hiroshi, Zama-shi, Kanagawa 252-8583 (JP)
(74) Representative: Vossius & Partner Patentanwälte Rechtsanwälte mbB
(86) International application number: PCT/JP2018/044728
(87) International publication number: WO 2019/124075

(57) **Abstract**

The present invention provides a method of concentrating a cyclolanostane compound and a lophenol compound in an aloe powder. In the present invention, the aloe powder is produced by soaking aloe mesophyll in hot water and drying and pulverizing the aloe mesophyll recovered from the hot water.

## Description

### TECHNICAL FIELD

The present invention relates to a method of producing an aloe powder. More specifically, it relates to a method of concentrating a phytosterol as an aloe-specific functional component during pulverization of the aloe mesophyll.

### BACKGROUND ART

Aloe is a general term for succulent plants belonging to the genus Aloe, reportedly including 300 or more species. Of these, Aloe vera is a plant which has been used in a folk remedy for external and internal applications for many years. Recently, the functionality possessed by aloe is drawing attention, and use of aloe for a functional food, a supplement, a medicine, a cosmetic, and the like is being studied.

A phytosterol is known as a functional component included in aloe. In particular, cyclolanostane compounds (9,19-cyclolanostane-3-ol and 24-methylene-9,19-cyclolanostane-3-ol) and lophenol compounds (4-methylcholest-7-en-3-ol, 4-methylergost-7-en-3-ol, and 4-methylstigmast-7-en-3-ol)) are specific to aloe, and various functionalities as represented by an anti-diabetic effect (Non Patent Literature 1) have been found in these compounds.

Patent Literature 1 discloses a purification method using supercritical extraction as a method of extracting the cyclolanostane compound and the lophenol compound included in aloe.

Further, as a method of extracting the phytosterol, for example, Patent Literature 2 discloses a method of recovering the phytosterol from rapeseed oil and soybean oil using methanol and a method of isolating the phytosterol by soaking the crude phytosterol in an organic solvent, followed by separation of the organic solvent.

Further, as a method of producing an aloe powder, Patent Literature 3 describes a method in which an aloe mesophyll solution is enzymatically digested using a polysaccharide digestive enzyme and a retentate fraction is recovered by performing membrane filtration using a micro filtration membrane or an ultrafiltration membrane and discloses that this method makes it possible to obtain aloe extract containing β-sitosterol, a phospholipid, and a dietary fiber. Further, Patent Literature 3 describes, in Example, that the cyclolanostane compound and the lophenol compound can be concentrated by about 1.5 times.

On the other hand, a polysaccharide included in the aloe powder is found to have various functionalities such as an anti-inflammatory effect (Patent Literature 4).

Patent Literature 5 discloses a method of producing the aloe powder characterized by having an aloin content of 25 ppm or less and a molecular weight of a contained polysaccharide of 3,000,000 Dalton or more by soaking Aloe vera gel in water or an extremely diluted aqueous solution.

### CITATION LIST

### PATENT LITERATURE

Patent Literature 1: JP 4095115 B2
Patent Literature 2: JP 4796695 B2
Patent Literature 3: JP 6066537 B2
Patent Literature 4: JP 2005-524617 A
Patent Literature 5: JP 4871735 B2

Non-Patent Literature 1: Biol.Pharm.Bull., 2006,29,1418-1422

### SUMMARY OF INVENTION

### TECHNICAL PROBLEM

The method of concentrating the cyclolanostane compound and the lophenol compound described in Patent Literature 1 imposes problems of requiring a huge apparatus and causing a large amount of costs for processing.

The method of concentrating the phytosterol described in Patent Literature 3 is effective in terms of concentrating the cyclolanostane compound and the lophenol compound. However, the method requires steps such as the enzymatic treatment and the filtration, leading to a demand for a simpler concentration method. Further, when the polysaccharide is enzymatically digested as in Patent Literature 3, the molecular weight of the polysaccharide becomes excessively low depending on the condition of the enzymatic treatment, sometimes making it difficult to obtain desired properties or functionalities derived from the aloe mesophyll.

Further, although the methods described in Patent Literatures 2 and 5 do not focus on the concentration of the cyclolanostane compound and the lophenol compound, the method of extracting the phytosterol using an organic solvent as in Patent Literatures 2 causes a problem of, for example, putting a burden on a worker from the standpoint of handling the organic solvent.

Although an application of the cyclolanostane compound and the lophenol compound included in aloe to a functional food and the like is increasingly expected, a conventional method of purifying the cyclolanostane compound and the lophenol compound generally requires the use of a special apparatus, agent, and solvent, thus the application to a food and the like, in particular, is limited.

Further, the phytosterol is a wax-like semi-solid material at normal temperature and is thus insoluble. This leads to difficulty in processing of a raw material obtained by purifying the phytosterol to a medicine and a food.

Thus, it is an object of the present invention to provide an aloe powder which includes a cyclolanostane compound and a lophenol compound at a high concentration and is suitable for food processing.

Further, it is another object of the present invention to provide an aloe powder which includes a cyclolanostane compound and a lophenol compound at a high concentration and also includes glucomannan.

### SOLUTION TO PROBLEM

The present inventors have found, as a result of studies, that a phytosterol, including a cyclolanostane compound and a lophenol compound, can be concentrated by soaking the aloe mesophyll of which the skin is peeled off in hot water and then recovering and pulverizing the aloe mesophyll, thereby completing the following invention.

That is, to solve the aforementioned object, the present invention is a method of producing an aloe powder including a phytosterol, including a hot water soaking step of soaking aloe mesophyll in hot water, a recovery step of recovering the aloe mesophyll soaked in the hot water, and a powdering step of drying and pulverizing the recovered aloe mesophyll.

According to the method of producing the aloe powder of the present invention, it becomes possible to easily produce the aloe powder which contains, at a high concentration, the phytosterol including the cyclolanostane compound and the lophenol compound. Further, according to the method of producing the aloe powder of the present invention, it becomes possible to produce the aloe powder sufficiently containing polysaccharides mainly composed of glucomannan included in aloe. Further, according to the production method of the present invention, it becomes possible to obtain the aloe powder that is excellent in water dispersibility and suitable for processing to a medicine, a food, a cosmetic, and the like.

According to a preferred embodiment of the present invention, the hot water is preferably 70°C or higher.

The cyclolanostane compound and the lophenol compound can be efficiently concentrated by keeping the temperature of the hot water within the aforementioned range.

According to a preferred embodiment of the present invention, in the hot water soaking step, the aloe mesophyll is soaked in the hot water in an amount 10 times by mass or more of the aloe mesophyll.

The cyclolanostane compound and the lophenol compound can be efficiently concentrated by maintaining the amount of the hot water within the aforementioned range.

According to a preferred embodiment of the present invention, prior to the hot water soaking step, there is a cutting step of cutting the aloe mesophyll to a maximum length of 30 mm or less.

The cyclolanostane compound and the lophenol compound can be efficiently concentrated by cutting the aloe mesophyll within the aforementioned range prior to the hot water soaking step.

According to a preferred embodiment of the present invention, the phytosterol includes a cyclolanostane compound and a lophenol compound and the total amount of both compounds is 80 µg or more with respect to 1 g of the aloe powder.

According to a preferred embodiment of the present invention, the aloe powder to be produced includes glucomannan and the amount of the glucomannan is 40 mg or more with respect to 1 g of the aloe powder.

In addition, to solve the aforementioned object is the aloe powder produced by the production method according to the present invention.

The aloe powder of the present invention includes the cyclolanostane compound and the lophenol compound at a high concentration in a tissue of the aloe mesophyll. Further, the aloe powder of the present invention includes a sufficient amount of glucomannan. Further, the aloe powder of the present invention, which is excellent in water dispersibility, can be easily processed to a medicine, a food, a functional food, a cosmetic, and the like.

In addition, to solve the aforementioned object, the present invention is the aloe powder prepared by drying and pulverizing the aloe mesophyll, in which the aloe powder includes the glucomannan in an amount of 40 mg or more and also the cyclolanostane compound and the lophenol compound in a total amount of 80 µg or more with respect to 1 g of the aloe powder.

The aloe powder of the present invention includes glucomannan, as well as the cyclolanostane compound and the lophenol compound, at a high concentration and is also excellent in processability. Thus, it is useful as a raw material of a functional food and the like.

### ADVANTAGEOUS EFFECTS OF INVENTION

According to the present invention, it becomes possible to provide the method of easily concentrating the phytosterol, particularly the cyclolanostane compound and the lophenol compound, in the aloe powder. Further, according to the present invention, it becomes possible to provide the aloe powder which contains large amounts of the cyclolanostane compound and the lophenol compound, as well as glucomannan, and which can be easily processed to a food and the like.

### DESCRIPTION OF EMBODIMENTS

Next, preferred embodiments of the present invention will be described in detail. However, the present invention is not limited to the following preferred embodiments, and any changes can be made within the scope of the present invention. Note that percentages used in the present specification are represented by mass unless otherwise noted.

The method of producing according to the present invention includes a hot water soaking step of soaking aloe mesophyll in hot water, a recovery step of recovering the aloe mesophyll soaked in the hot water, and a powdering step of drying and pulverizing the recovered aloe mesophyll.

Further, a preferred embodiment includes, prior to the hot water soaking step, a cutting step of cutting the aloe mesophyll.

### < 1> Method of producing aloe powder

### (1) Aloe mesophyll

The aloe mesophyll used as a raw material in the production method of the present invention is the mesophyll obtained from a plant belonging to the genus Aloe, preferably the mesophyll of Aloe vera.

As the aloe mesophyll, the skin of a raw leaf of aloe is peeled off, and the mesophyll part is removed from the raw leaf and can be used. Alternatively, a raw leaf of aloe is cut, and the resulting raw leaf in which the mesophyll part is exposed may be used.

Further, the mesophyll surface is washed as needed. A component such as anthraquinone can be removed by washing the mesophyll surface. Further, removing a viscous substance on the surface facilitates cutting when the cutting step described below is performed. The washing can be performed, for example, by a method in which the aloe mesophyll is washed with water having normal temperature (from 5 to 35°C).

### (2) Cutting step

The cutting step is a step of cutting the aloe mesophyll and preferably performed prior to the hot water soaking step. Cutting makes it possible to efficiently concentrate the cyclolanostane compound and the lophenol compound.

It is preferable that, as a size of the cut piece, the length is 30 mm or less, preferably 15 mm or less. A lower limit value of the size of the cut piece is not particularly limited. However, the maximum length is set to 10 mm as a standard of the lower limit value. Note that the cutting in the present invention is performed such that a tissue of the aloe mesophyll is maintained in the cut piece. Performing the cutting in a state of maintaining the tissue of the aloe mesophyll in this manner allows the cyclolanostane compound and the lophenol compound, preferably glucomannan as well, to be kept in the tissue of the aloe mesophyll, while allowing other components to be efficiently removed, in the following hot water soaking step.

The cutting method is not particularly limited. However, a method of pressing the aloe mesophyll into a slit can be used.

Note that, in the case of using the method of pressing the aloe mesophyll into the slit, the size of the cut piece described above can be considered the same as the size of a slit width.

### (3) Hot water soaking step

The hot water soaking step is a step in which the aloe mesophyll, preferably having already been subjected to the cutting step, is soaked in hot water. The hot water in the present invention has a temperature of higher than 60°C, preferably 70°C or higher.

The temperature of the hot water is, for example, 80°C or higher, 85°C or higher, or 90°C or higher. This makes it possible to efficiently concentrate the cyclolanostane compound and the lophenol compound in the aloe mesophyll. Further, the upper limit of the temperature of the hot water is preferably 100°C. The upper limit of 100°C makes it possible to obtain the aloe powder having excellent appearance in which browning is reduced.

The soaking time is not particularly limited. However, it is preferably 2 minutes or more.

Examples of a combination of the soaking temperature and the soaking time preferably include the followings.

From 95°C to 100°C for preferably from 1 to 5 minutes, more preferably from 2 to 4 minutes, particularly preferably from 2 to 3 minutes.

In a preferred embodiment, the upper limit of the soaking time is set from the standpoint of reducing browning.

Further, regarding a quantity ratio (mass) of the aloe mesophyll and the hot water in the hot water soaking step, the hot water is preferably 10 times by mass or more, more preferably 15 times by mass or more, further more preferably 20 times by mass or more, with respect to the aloe mesophyll. Maintaining such a range can improve the concentration efficiency. The upper limit of the quantity ratio of the hot water can be set, as a standard, 30 of the hot water with respect to 1 of the aloe mesophyll considering the productivity including the water treatment.

### (4) Recovery step

The aloe mesophyll soaked in the hot water is recovered from the hot water. The recovery may be performed, for example, by scooping the aloe mesophyll with a sieve made of stainless steel or the like.

### (5) Powdering step

The recovered aloe mesophyll was dried and pulverized. The pulverization can be performed during drying. The drying method is not particularly limited. However, hot air drying, fluidized bed drying, spray drying, drum drying, low temperature drying, vacuum freeze drying, pressure drying, or the like can be employed. Further, the pulverization method is not particularly limited. However, impact crushing, airflow crushing, freeze crushing, or the like can be employed.

### <2> Aloe powder

The aloe powder of the present invention can be produced by the method of producing the aloe powder of the present invention described above. The aloe powder produced by the method of producing the aloe powder of the present invention has a structure in which the cyclolanostane compound and the lophenol compound are retained in the tissue mainly composed of polysaccharides included in the aloe powder.

Hereinafter, preferred embodiments of the aloe powder will be described.

Regarding the content of the cyclolanostane compound and the lophenol compound in the aloe powder, the total amount of both compounds is 80 µg or more, preferably 100 µg or more, more preferably 110 µg or more, particularly preferably 120 µg or more, with respect to 1 g of the aloe powder. An upper limit value is about 200 µg as a standard.

The content of glucomannan in the aloe powder is 40 mg or more, preferably 50 mg or more, more preferably 55 mg or more, with respect to 1 g of the aloe powder. An upper limit value is about 180 mg as a standard.

Further, the average molecular weight of glucomannan is preferably from 800 to 1,250,000 Dalton, more preferably from 25,000 to 950,000 Dalton.

The aloe powder obtained by the production method of the present invention and the aloe powder of the present invention having the aforementioned composition can be used as a raw material of a medicine, a food, a functional food, a cosmetic, and the like.

The aloe powder of the present invention is excellent in water dispersibility as the hydrophobic cyclolanostane compound and lophenol compound are held in the tissue mainly composed of polysaccharides included in the aloe powder. Thus, the aloe powder is preferably used as a raw material of a food such as a beverage and jelly and a functional food.

### EXAMPLES

Hereinafter, the present invention will be described further in detail by way of the following Examples. However, the present invention is not limited to these Examples.

### [1] Test 1

The present test was performed to validate of the effect of the hot water soaking step on the concentration of the cyclolanostane compound and the lophenol compound.

### <Example 1>

The mesophyll of Aloe vera (hereinafter, aloe mesophyll), of which the skin was peeled off, in an amount of 328.8 kg was washed and then pressed into a slit (a slit width of 15 mm) to cut the aloe mesophyll into a cubical shape having a length of one side of from 10 to 15 mm. Next, the cut aloe mesophyll in an amount of 25 kg was soaked in 600 kg of hot water (96°C) for 2.5 minutes. Next, the soaked aloe mesophyll was dried by hot air at 65°C for 8 hours using a shelf dryer, and then the dried aloe mesophyll was pulverized using an airflow mill to obtain the aloe powder in an amount of 1.43 kg.

### <Comparative example 1>

The aloe mesophyll, of which the skin was peeled off, in an amount of 20 kg was washed and then dried by hot air at 65°C for 8 hours using a shelf dryer. The dried aloe mesophyll was pulverized using an airflow mill to obtain the aloe powder in an amount of 150 g (without the cutting step and the hot water soaking step).

### <Comparative example 2>

The aloe mesophyll, of which the skin was peeled off, in an amount of 16.5 kg was washed and then pressed into a slit to cut the aloe mesophyll into a cubic shape having a length of one side of from 10 to 15 mm. Next, the cut aloe mesophyll was dried by hot air at 65°C for 8 hours using a shelf dryer, and then the dried aloe mesophyll was pulverized using an airflow mill to obtain the aloe powder in an amount of 136 g (with the cutting step but without the hot water soaking step).

The total value of two kinds of the cyclolanostane compounds (9,19-cyclolanostane-3-ol and 24-methylene-9,19-cyclolanostane-3-ol) and three kinds of the lophenol compounds (4-methylcholest-7-en-3-ol, 4-methylergost-7-en-3-ol, and 4-methylstigmast-7-en-3-ol) (hereinafter, also simply referred to as "phytosterol total value") in Example and Comparative examples was quantified by LC-MS (manufactured by Shimadzu Corp.) on the basis of the calibration curve created using brassicasterol (manufactured by Wako Pure Chemical Industries, Ltd.) as an internal standard substance. The result is shown in Table 1.

### [Table 1]

**Table 1. Difference in phytosterol total value by presence/absence of cutting step and hot water soaking step**

| | Cutting step | Hot water soaking step | Phytosterol total value (µg/g) |
|---|---|---|---|
| Example 1 | Yes (10 to 15 mm) | Yes | 126.1 |
| Comparative example 1 | No | No | 62.5 |
| Comparative example 2 | Yes (10 to 15 mm) | No | 77.1 |

As is evident from Table 1, the aloe powder in Example 1 produced by the production method including the hot water soaking step had the significantly higher phytosterol total value (from 1.6 to 2 times) as compared with the aloe powders in Comparative example 1 and Comparative example 2 produced by the production methods without including the hot water soaking step.

When the phytosterol total value was compared between Comparative example 1 and Comparative example 2, Comparative example 2 in which the cutting step was performed had the higher phytosterol total value than the Comparative example 1 in which the cutting step was not performed. However, an increase in concentration caused by the hot water soaking (Example 1 and Comparative example 2) was more significant than an increase in concentration caused by the cutting (Comparative example 2 and Comparative example 1).

These results showed that the hot water soaking step was extremely important for the concentration of the cyclolanostane compound and the lophenol compound. Further, it was shown that the cyclolanostane compound and the lophenol compound could be efficiently concentrated by combining the cutting step and the hot water soaking step.

### [2] Test 2

The present test was performed to validate of the effect of the degree of the cutting (the size of the cut piece subjected to the hot water soaking) in the cutting step on the concentration of the cyclolanostane compound and the lophenol compound.

### <Example 2>

The aloe mesophyll, of which the skin was peeled off, in an amount of 1 kg was washed and then pressed into a slit (a slit width of 25 mm) to cut the aloe mesophyll into a cubic shape having a length of one side of from 20 to 25 mm. Next, the cut aloe mesophyll was soaked in 24 kg of hot water (96°C) for 2.5 minutes. Next, the soaked aloe mesophyll was dried by hot air at 65°C for 8 hours using a shelf dryer, and then the dried aloe mesophyll was pulverized using an airflow mill to obtain the aloe powder in an amount of 8 g (with the cutting step (20 to 25 mm) and with the hot water soaking step).

The phytosterol total value was quantified in the same manner as in Test 1. The result thereof, as well as the result of Example 1 in Test 1, are shown in Table 2.

### [Table 2]

**Table 2. Difference in phytosterol total value by the degree of the cutting**

| | Cutting step | Hot water soaking step | Phytosterol total value (µg/g) |
|---|---|---|---|
| Example 1 | Yes (10 to 15 mm) | Yes | 126.1 |
| Example 2 | Yes (20 to 25 mm) | Yes | 117.0 |

As is evident from Table 2, it was shown that the phytosterol could be efficiently concentrated by adjusting the size of the cut piece to from 10 to 25 mm in the cutting step. Further, it was shown that the concentration efficiency increased by adjusting the size of the cut piece to from 10 to 15 mm, in particular.

### [Test 3]

The present test was performed to validate of the effect of the quantity ratio of the aloe mesophyll and the hot water in the hot water soaking step on the concentration of the cyclolanostane compound and the lophenol compound.

### <Example 3>

The aloe mesophyll, of which the skin was peeled off, in an amount of 600 g was washed and then pressed into a slit to cut the aloe mesophyll into a cubic shape having a length of one side of from 10 to 15 mm. Next, the cut aloe mesophyll was soaked in 6 kg of hot water (96°C) for 2.5 minutes. Next, the soaked aloe mesophyll was dried by hot air at 65°C for 8 hours using a shelf dryer, and then the dried aloe mesophyll was pulverized using an airflow mill to obtain the aloe powder in an amount of 2.4 g (with the cutting step and with the hot water soaking step (aloe mesophyll : hot water = 1:10).

The phytosterol total value was quantified in the same manner as in Test 1. The result thereof, as well as the result of Example 1 and Comparative example 2 in Test 1, are shown in Table 3.

### [Table 3]

**Table 3. Difference in phytosterol total value by the difference of the quantity ratio of the aloe mesophyll and the hot water**

| | Cutting step | Hot water soaking step | Phytosterol total value (µg/g) |
|---|---|---|---|
| Example 3 | Yes | Yes (aloe mesophyll : hot water = 1:10) | 88.6 |
| Example 1 | Yes | Yes (aloe mesophyll : hot water = 1:24) | 126.1 |
| Comparative Example 2 | Yes | No | 77.1 |

As is evident from Table 3, it was shown that, regarding the quantity ratio (mass) of the aloe mesophyll and the hot water, the hot water was preferably 10 or more with respect to 1 of the aloe mesophyll. Further, it was shown that the concentration efficiency significantly increased when the hot water was preferably 20 or more with respect to 1 of the aloe mesophyll.

### [Test 4]

The present test was performed to validate of the effect of the temperature of the hot water in the hot water soaking step on the concentration of the cyclolanostane compound and the lophenol compound.

### <Example 4>

The aloe mesophyll, of which the skin was peeled off, in an amount of 1 kg was washed and then pressed into a slit to cut the aloe mesophyll into a cubic shape having a length of one side of from 10 to 15 mm. Next, the cut aloe mesophyll was soaked in 24 kg of hot water (90°C) for 2.5 minutes. Next, the soaked aloe mesophyll was dried by hot air at 65°C for 8 hours using a shelf dryer, and then the dried aloe mesophyll was pulverized using an airflow mill to obtain the aloe powder in an amount of 7.8 g (with the cutting step and with the hot water soaking step (90°C)).

### <Comparative Example 3>

The aloe mesophyll, of which the skin was peeled off, in an amount of 600 g was washed and then pressed into a slit to cut the aloe mesophyll into a cubic shape having a length of one side of from 10 to 15 mm. Next, the cut aloe mesophyll was soaked in 14.4 kg of hot water (60°C) for 2.5 minutes. Next, the soaked aloe mesophyll was dried by hot air at 65°C for 8 hours using a shelf dryer, and then the dried aloe mesophyll was pulverized using an airflow mill to obtain the aloe powder in an amount of 2.5 g (with the cutting step and with the hot water soaking step (60°C)).

The phytosterol total value was quantified in the same manner as in Test 1. The result thereof, as well as the result of Example 1 in Test 1, are shown in Table 4.

### [Table 4]

**Table 4. Difference in phytosterol total value by the difference of the temperature of the hot water**

| | Cutting step | Hot water soaking step | Phytosterol total value (µg/g) |
|---|---|---|---|
| Example 4 | Yes | Yes (90°C) | 120.0 |
| Comparative Example 3 | Yes | Yes (60°C) | 78.2 |
| Example 1 | Yes | Yes (96°C) | 126.1 |

As is evident from Table 4, it was shown that, regarding the temperature of water, the temperature of the hot water needed to be higher than 60°C. A significant concentration effect could be obtained by using the hot water of 90°C or higher.

Note that, as Example, a test was performed under the following conditions: an acidulant such as sodium chloride and citric acid was dissolved in the hot water, the temperature of the hot water was increased to 100°C or higher, and the soaking in the hot water was performed for 2.5 minutes. Under such conditions, although the phytosterol total value was high, the obtained aloe powder showed browning.

Thus, the upper limit of the hot water temperature was preferably 100°C from the standpoint of appearance of the product.

### [Test 5]

The present test was performed to validate of the effect of the soaking time in the hot water soaking step on the concentration of the cyclolanostane compound and the lophenol compound.

### <Example 5>

The aloe mesophyll, of which the skin was peeled off, in an amount of 2049 kg was cut into a cubic shape having a length of one side of from 10 to 15 mm. Next, the cut cube in an amount of 25 kg was soaked in 600 kg of hot water (96°C) for 4 minutes. Subsequently, the aloe mesophyll was dried using a shelf dryer to obtain the aloe powder in an amount of 6.8 kg (with the cutting step and the hot water soaking step (96°C, 4 minutes)).

The phytosterol total value was quantified in the same manner as in Test 1. The result thereof, as well as the result of Example 1 in Test 1, are shown in Table 5.

### [Table 5]

**Table 5. Difference in phytosterol total value by the difference of the soaking condition**

| | Cutting step | Hot water soaking step | Phytosterol total value (µg/g) |
|---|---|---|---|
| Example 5 | Yes | Yes (96°C, 4 minutes) | 124.30 |
| Example 1 | Yes | Yes (96°C, 2.5 minutes) | 126.12 |

As is evident from Table 5, the conditions of the hot water temperature of 96°C and the soaking time of 4 minutes showed almost the same concentration effect as the conditions of the hot water temperature of 96°C and the soaking for 2.5 minutes.

The present Example showed that the phytosterol could be concentrated in a very short time of within 2 minutes.

### [Test 6] Measurement of glucomannan amount

Glucomannan included in the aloe powder obtained in Example 1 was measured. The measurement was performed by a method described in J. AOAC Int., 2005, 88, 684-691. The result is shown in Table 6.

### [Table 6]

**Table 6. Amount of glucomannan included in the aloe powder obtained in Example 1**

| | Cutting step | Hot water soaking step | Amount of glucomannan (mg/g) |
|---|---|---|---|
| Example 1 | Yes | Yes (96°C) | 58.2 |

The result of the present test showed that the aloe powder obtained by the production method of the present invention included a sufficient amount of glucomannan.

This showed that the method of the present invention could concentrate the cyclolanostane compound and the lophenol compound while maintaining glucomannan included in natural aloe.

### [Test 7]

The present test was performed to validate of the effect of the temperature of the hot water in the hot water soaking step on the concentration of the cyclolanostane compound and the lophenol compound.

### <Example 6>

The aloe mesophyll of which the skin was peeled off in an amount of 600 g was washed and then pressed into a slit to cut the aloe mesophyll into a cubic shape having a length of one side of from 10 to 15 mm. Next, the cut aloe mesophyll was soaked in 14.4 kg of hot water (70°C) for 2.5 minutes. Next, the soaked aloe mesophyll was dried by hot air at 65°C for 8 hours using a shelf dryer, and then the dried aloe mesophyll was pulverized using an airflow mill to obtain the aloe powder in an amount of 2.2 g (with the dicing step and with the hot water soaking step (70°C)).

### <Example 7>

The aloe mesophyll, of which the skin was peeled off, in an amount of 600 g was washed and then pressed into a slit to cut the aloe mesophyll into a cubic shape having a length of one side of from 10 to 15 mm. Next, the cut aloe mesophyll was soaked in 14.4 kg of hot water (80°C) for 2.5 minutes. Next, the soaked aloe mesophyll was dried by hot air at 65°C for 8 hours using a shelf dryer, and then the dried aloe mesophyll was pulverized using an airflow mill to obtain the aloe powder in an amount of 2.5 g (with the dicing step and with the hot water soaking step (80°C)).

The phytosterol total value was quantified in the same manner as in Test 1. The result thereof, as well as the result of Example 4 and Comparative example 3 in Test 4, are shown in Table 7.

### [Table 7]

**Table 7. Difference in phytosterol total value by the difference of the soaking condition**

| | Cutting step | Hot water soaking step | Phytosterol total value (µg/g) |
|---|---|---|---|
| Comparative Example 3 | Yes | Yes (60°C) | 78.2 |
| Example 6 | Yes | Yes (70°C) | 119.6 |
| Example 7 | Yes | Yes (80°C) | 124.0 |
| Example 4 | Yes | Yes (90°C) | 120.0 |

As is evident from Table 7, it was shown that there was a significant difference in the extraction efficiency between Comparative example 3 using the water at 60°C and Example 6 using the hot water at 70°C. Further, it was shown that there was no significant difference in the concentration efficiency in a range from 70 to 90°C. This showed that using the hot water at 70°C or higher could increase the concentration efficiency of the phytosterol.

### <Production example 1>

The aloe powder was produced by the method described in Example 1, and tablet confectionery was produced on the basis of a composition described in Table 8. Each powder of 20 g of the aloe powder, 54 g of reduced maltose, 10 g of cellulose, 5 g of acidulant, 5 g of an emulsifier, 4 g of a flavoring agent, 1.5 g of tricalcium phosphate, and 0.5 g of sweetener was combined and uniformly mixed, and a tableting machine was used to continuously compact the above mixed powder under a pressure of 9.8 KPa at a tableting speed of 12 tablets per minute to produce 5000 tablets (about 1000 g) of the tablet confectionery each tablet weighing 200 mg.

In the production, each component could be uniformly mixed and direct tableting could be performed without the need for a pretreatment such as granulation. The appearance of the produced tablet confectionery had no noticeable browning.

### [Table 8]

**Table 8. Composition of tablet confectionery**

| Raw material name | Composition (% by mass) |
|---|---|
| aloe powder | 20 |
| reduced maltose | 54 |
| cellulose | 10 |
| acidulant | 5 |
| emulsifier | 5 |
| flavoring agent | 4 |
| tricalcium phosphate | 1.5 |
| sweetener | 0.5 |

### <Production example 2>

The aloe powder was produced by the method described in Example 1 and jelly was produced on the basis of a composition described in Table 9. Each raw material of 25 g of the aloe powder, 120 g of erythritol, 50 g of an emulsifier, 42 g of muscat fruit juice, 15 g of tricalcium phosphate, 13 g of a gelling agent, 10 g of a flavoring agent, 8 g of sweetener, 2 g of acidulant, and 715 g of water was combined and uniformly mixed. After being sterilized at 93°C for 1 minute, the mixture in an amount of 20 g was filled in an aluminum stick to produce 50 units (about 1000 g) of the jelly.

In the production, each component could be uniformly mixed and the production could be completed without the need for a pretreatment such as special emulsification. The appearance of the produced jelly had no noticeable browning.

### [Table 9]

**Table 9. Composition of jelly**

| Raw material name | Composition (% by mass) |
|---|---|
| aloe powder | 2.5 |
| erythritol | 12 |
| emulsifier | 5 |
| muscat fruit juice | 4.2 |
| tricalcium phosphate | 1.5 |
| gelling agent | 1.3 |
| flavoring agent | 1.0 |
| sweetener | 0.8 |
| acidulant | 0.2 |
| water | 71.5 |

### <Production example 3>

The aloe powder was produced by the method described in Example 1 and a drink was produced on the basis of a composition described in Table 10. Each raw material of 10 g of the aloe powder, 60 g of erythritol, 4.9 g of acidulant, 4 g of a flavoring agent, 2 g of an emulsifier, 0.1 g of sweetener, and 919 g of water was combined and uniformly mixed. After being sterilized at 80°C for 30 minutes, the mixture in an amount of 50 g was filled in a brown glass bottle to produce 20 drinks (about 1000 g).

In the production, each component could be uniformly mixed and the production could be completed without the need for a pretreatment such as special emulsification. The produced drink had no precipitate or the like, or noticeable browning.

### [Table 10]

**Table 10. Composition of drink**

| Raw material name | Composition (% by mass) |
|---|---|
| aloe powder | 1 |
| erythritol | 6 |
| acidulant | 0.49 |
| flavoring agent | 0.4 |
| emulsifier | 0.2 |
| sweetener | 0.01 |
| water | 91.9 |

### INDUSTRIAL APPLICABILITY

According to the present invention, it becomes possible to obtain the aloe powder which includes the cyclolanostane compound and the lophenol compound at a high concentration while maintaining glucomannan. Thus, the aloe powder of the present invention is useful as a raw material in technical fields such as a medicine, a food, a functional food, a cosmetic, and the like.

## Claims

1. A method of producing an aloe powder including a phytosterol, comprising:
a hot water soaking step of soaking aloe mesophyll in hot water;
a recovery step of recovering the aloe mesophyll soaked in the hot water; and
a powdering step of drying and pulverizing the recovered aloe mesophyll.

2. The method of producing the aloe powder according to claim 1, wherein the hot water is 70°C or higher.

3. The method of producing the aloe powder according to claim 1 or 2, wherein, in the hot water soaking step, the aloe mesophyll is soaked in the hot water in an amount 10 times by mass or more of the aloe mesophyll.

4. The method of producing the aloe powder according to any of claims 1 to 3, comprising, prior to the hot water soaking step, a cutting step of cutting the aloe mesophyll to a maximum length of 30 mm or less.

5. The method of producing the aloe powder according to any of claims 1 to 4, wherein the phytosterol includes a cyclolanostane compound and a lophenol compound and a total amount of both compounds is 80 µg or more with respect to 1 g of the aloe powder.

6. The method of producing the aloe powder according to any of claims 1 to 5, wherein the aloe powder includes glucomannan and a content of the glucomannan is 40 mg or more with respect to 1 g of the aloe powder.

7. The aloe powder produced by the production method according to any of claims 1 to 6.

8. The aloe powder prepared by drying and pulverizing the aloe mesophyll, wherein the aloe powder includes the glucomannan in an amount of 40 mg or more and also the cyclolanostane compound and the lophenol compound in a total amount of 80 µg or more with respect to 1 g of the aloe powder.
